# FASCICULE DE BREVET EUROPEEN

(11) **EP 1 799 225 B1**
(45) Date de publication et mention de la délivrance du brevet: **07.12.2011**
(21) Numéro de dépôt: 05809765.0
(22) Date de dépôt: 14.10.2005
(51) Int. Cl.: A61K 31/55, A61P 11/12

(54) **UTILISATION DE DERIVES DE PAULLONES POUR LA FABRICATION DE MEDICAMENTS POUR LE TRAITEMENT DE LA MUCOVISCIDOSE ET DE MALADIES LIEES A DEFAUT D'ADRESSAGE DES PROTEINES DANS LES CELLULES**
VERWENDUNG VON PAULLON-DERIVATEN ZUR HERSTELLUNG VON MEDIKAMENTEN ZUR BEHANDLUNG VON MUKOVISZIDOSE UND KRANKHEITEN AUFGRUND VON PROTEIN-ADRESSIERUNGSFEHLERN IN ZELLEN
USE OF PAULLONE DERIVATES FOR THE PRODUCTION OF MEDICAMENTS FOR THE TREATMENT OF MUCOVISCIDOSIS AND DISEASES RELATED TO PROTEIN ADDRESSING ERRORS IN CELLS

(30) Priorité: 15.10.2004 FR 0410961
(43) Date de publication de la demande: 27.06.2007
(73) Titulaire: CENTRE NATIONAL DE LA RECHERCHE SCIENTIFIQUE (CNRS), 75794 Paris Cedex 16 (FR); UNIVERSITE DE POITIERS, 86034 Poitiers Cedex (FR)
(72) Inventeur: BECQ, Frédéric, F-86280 Poitiers (FR); MEIJER, Laurent, F-29680 Roscoff (FR); KUNICK, Conrad, 21149 Hamburg (DE)
(74) Mandataire: Novagraaf Technologies
(86) Numéro de dépôt international: PCT/FR2005/002556
(87) Numéro de publication internationale: WO 2006/042948

(56) Documents cités:
- WO-A-00/24391
- FR-A- 2 804 959

## Description

L'invention vise l'utilisation de kenpaullone et de dérivés de la kenpaullone pour fabriquer des médicaments capables de restaurer l'adressage de protéines du réticulum endoplasmique vers les membranes plasmiques. Elle vise tout particulièrement le traitement de la mucoviscidose.

La mucoviscidose (CF : *Cystic Fibrosis*) est la maladie génétique récessive, autosomique, létale la plus répandue dans les populations européennes et nord américaines. Le gène CF (locus 7q31) code pour la protéine trans-membranaire nommée CFTR (pour *Cystic Fibrosis Transmembrane Conductance Regulator*). Des mutations du gène CF provoquent un transport anormal d'eau et d'électrolytes à travers les membranes cellulaires de divers organes comme les poumons, les glandes sudoripares, l'intestin, et le pancréas exocrine. Bien qu'il existe plus de 1000 mutations de la protéine CFTR, la mutation la plus fréquente (70 % des patients) est la délétion d'une phénylalanine dans le domaine NBF1 en position 508 (delF508). La principale cause de mortalité des patients CF est liée à cette délétion et conduit à des infections ou à une insuffisance pulmonaire provoquées par une augmentation de la viscosité du mucus. Cette viscosité entraîne l'occlusion des voies respiratoires et favorise les infections par les bactéries opportunistes. Une aggravation est de plus constatée au niveau digestif et notamment pancréatique (patient pancréatique-insuffisant). La protéine CFTR est une glycoprotéine de 1480 acides aminés, appartenant à la superfamille des transporteurs membranaires ABC. CFTR est un canal chlorure localisé dans la membrane plasmique apicale des cellules épithéliales pulmonaires chez les individus sains.

CFTR est responsable du transport transépithélial d'eau et d'électrolytes et permet chez un individu sain l'hydratation des voies aériennes pulmonaires.

Chez les patients CF, homozygotes pour la mutation delF508, et plus généralement pour les mutants de classe II (mutations produisant une protéine absente de la membrane plasmique), cette protéine est absente des membranes plasmiques du fait d'un mauvais adressage de la protéine qui est retenue dans le réticulum endoplasmique (RE). L'hydratation des voies aériennes, pulmonaires n'est plus fonctionnelle dans ce cas. La délétion delF508 perturbe le repliement du domaine NBF1 et empêche la maturation complète de la protéine qui est donc dégradée très tôt au cours de sa biosynthèse. Cependant, si la protéine delF508 atteint la membrane, elle fonctionne comme un canal chlorure.

Une des clés d'un traitement de cette maladie consiste donc en un ré-adressage de delF508 vers la membrane plasmique des cellules au niveau de laquelle l'activité de transport de delF508 peut être stimulée par des agonistes physiologiques.

De manière surprenante, les inventeurs ont mis en évidence que les dérivés de paullones connus notamment pour leur effet anti-prolifératif étaient capable d'activer le CFTR sauvage et des formes mutées et de provoquer une re-localisation membranaire de la protéine delF508-CFTR restaurant ainsi sa capacité de transport trans-membranaire. De manière générale, ces dérivés sont capables de restaurer un défaut d'adressage des protéines dans les cellules. De plus, ces composés présentent l'intérêt d'une innocuité élevée.

Le document WO 0024391 décrit une méthode de traitement de maladies, dont la Fibrose Cystique ou mucoviscidose, consistant à administrer un agent permettant la libération de protéines du réticulum endoplasmique. Il décrit comme agent des molécules de structures très diverses comme la thapsigargine, l'acide clopiazonique, le DBHQ (di-tert-butylhydroquinone), l'halothane ou des oligonucléotides antisense de UGGT, calnexin ou Ca²⁺-ATPase ainsi que des dérivés de ceux-ci.

D1 décrit que les dérivés de paullone inhibent l'enzyme GSK-3β et peuvent être utilisés pour le traitement du diabète. Or, l'inhibition de l'enzyme GSK-3β n'a aucun lien avec le traitement de la mucoviscidose. La glycogne synthase kinase-3 (GSK-3) est une protéine kinase importante impliquée dans la régulation du métabolisme du glucose dans les cellules. Ainsi, cette enzyme, bien que cible thérapeutique importante pour les maladies citées, n'est pas associée à la physiopathologie de la mucoviscidose ni aux mécanismes de régulations des fonctions respiratoires chez l'homme.

L'invention à donc pour but de fournir une nouvelle utilisation des dérivés de paullones pour fabriquer des médicaments pour le traitement de la mucoviscidose et de maladies liées à un défaut d'adressage des protéines dans les cellules.

Ces dérivés répondent notamment à la formule générale (II) : 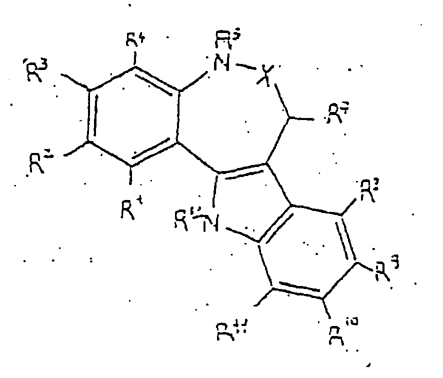 dans laquelle
- X représente un groupe C = O, C-S-CH₃, C-S, -C-NHOH;
- R⁵ représente un atome d'hydrogène ou un groupe alkyle en C₁ à C₅,
- R¹² représente un atome d'hydrogène, ou un groupe -
   C-CO₂-(CH₃)₃,
   dans laquelle,
- R¹ à R⁴, R⁷ à R¹¹, identiques ou différents, représentent un atome d'hydrogène, d'halogène (F, Cl, Br, I), un groupe hydroxy, alkylènehydroxy, alcynealkylènehydroxy, alcynehydroxycyclohexyle, alkyle, alkoxy, alkylènealkoxy, alkylènecyano, ces radicaux étant à chaîne droite ou ramifiée, en C1 à C18, le groupe alkylène étant saturé ou insaturé; ladite chaîne étant le cas échéant substituée par un ou plusieurs groupes hydroxy ou amino ; un groupe trifluorométhyle; un groupe -COM; -COOM; ou -CH₂COOM, (avec M représentant un atome d'hydrogène, un groupe alkyle en Cl à C18, à chaîne droite ou ramifiée, substituée le cas échéant par un ou plusieurs groupes hydroxy et/ou amino) ; un groupe nitroso; ou un groupe cyano ;
et les sels physiologiquement acceptables de ces dérivés.

Dans un groupe de dérivés de paullones de ces différentes familles, X représente C = 0.

L'invention vise plus spécialement une nouvelle utilisation de la kenpaullone pour fabriquer des médicaments pour le traitement de la mucoviscidose.

La kenpaullone utilisée conformément à l'invention répond à la formule (V) :

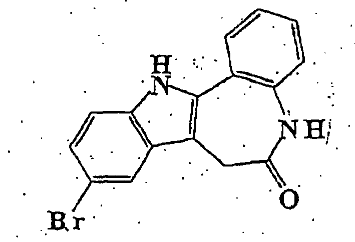

Comme illustré par les exemples, la kenpaullone, est particulièrement efficace pour provoquer la re-localisation membranaire de la protéine delF508-CFTR dans la mucoviscidose où cette protéine est retenue dans le réticulum endoplasmique, et de restaurer ainsi sa capacité de transport trans-membranaire.

Lors de l'élaboration des médicaments, les principes actifs, utilisés en quantités thérapeutiquement efficaces, sont mélangés avec les véhicules pharmaceutiquement acceptables pour le mode d'administration choisi.

Ainsi pour une administration par voie orale, les médicaments préparés sous forme de gélules, comprimés, dragées, capsules, pilules, gouttes, sirops et analogues. De tels médicaments peuvent renfermer de 1 à 100 mg de principe actif par .unité.

Pour l'administration par voie injectable (intraveineuse, sous-cutanée, intramusculaire), les médicaments se présentent sous.forme de solutions stériles ou stérilisables.

Ils peuvent être également sous forme de suspensions ou d'émulsions.

Les médicaments de l'invention sont plus particulièrement administrés sous forme d'aérosols.

Les doses par unité de prise peuvent varier de 1 à 50 mg de principe actif. La posologie quotidienne est choisie de manière à obtenir une concentration finale d'au plus 100 µM en dérivé de paullones dans le sang du patient traité.

D'autres caractéristiques et avantages de l'invention seront donnés dans les résultats rapportés ci-après afin d'illustrer l'invention.

Dans ces exemples, il est fait référence aux figures 1 à 5 qui représentent, respectivement :
- la figure 1 : un histogramme montrant lé pourcentage d'activation du CFTR après traitement de cellules CF15 dans différentes conditions,
- la figure 2A : le pourcentage d'activation du CFTR en fonction du log entre [kenpaullone] M et la figure 2B : l'effet d'inhibiteurs connus de CFTR sur l'activité delF508 après traitement par la kenpaullone,
- la figure 3A : une photo montrant la localisation de delF508 dans les compartiments intra-cellulaires et la figure 3B : une photo montrant la re-direction de la protéine vers la membrane, après traitement par la kenpaullone,
- les figures 4A à 4C : l'immunolocalisation de delF508-CFTR, après 2 h de traitement ou l'absence de traitement ;
- les figures 5A et 5B : l'activation de delF508-CFTR dans des cellules CF15 après traitement par la kenpaullone.

### MATERIEL ET METHODES

### M1. Culture cellulaire

Cellules CHO-WT : Les cellules CHO (Chinese Hamster Ovary) sont des fibroblastes qui ont été transfectés avec le gène du CFTR sauvage (CFTR-WT). Ces cellules vont donc surexprimer la protéine CFTR.

Milieu de culture : Milieu MEM alpha (GIBCO) + 7 % de sérum de veau foetal + 0,5 % de pénicilline/streptomycine + 100 µM de méthotrexate (améthoptérine, Sigma).

Cellules CF15 : Les cellules CF15 sont des cellules épithéliales humaines d'origine nasale qui expriment le gène ΔF508-CFTR.

Milieu de culture : Milieu DMEM + HAM F12 + 10 % de FCS + 0,6 % dé pénicilline/streptomycine + facteur de croissance (insuline 5 µg/ml, transferrine 5 µg/ml, épinéphrine 5,5 µM, adénine 0,18 mM, EGF 10 ng/ml, T3 2 nM, hydrocortisone 1,1 µM).

Cellules Calu-3 : Les cellules Calu-3 sont des cellules épithéliales humaines d'origine pulmonaire qui expriment le gène du CFTR sauvage.

Milieu de culture : Milieu DMEM/F12 avec glutamax + 7 % de sérum de veau foetal + 1 % de pénicilline/streptomycine.

### M2. Immunomarquage

L'immunomarquage permet de visualiser la localisation cellulaire de la protéine CFTR grâce à un anticorps (Ac) primaire anti-CFTR, puis un anticorps secondaire antianticorps primaire marqué au fluorophore Cy3.

Les cellules sont préalablement ensemencées sur des lamelles dans du milieu de culture approprié. 3 lavages au TBS (NaCl : 157 mM, Tris base : 20 µM, pH 7,4) de 5 min chacun sont effectués. Les cellules sont alors fixées, par ajout de TBS-paraformaldéhyde (3 %) pendant 20 min. Après 3 lavages au TBS (5 min), les cellules sont incubées avec du TBS-triton 0,1 % (10 min) qui permet la formation de trous dans la membrane cellulaire, puis 3 lavages au TBS sont à nouveau effectués avant la mise en présente des cellules avec le TBS-BSA 0,5 %-saponine 0,05 % pendant 1 h. Les cellules sont ensuite incubées avec l'anticorps primaire anti-C terminal CFTR (2 µg/ml) pendant 1 h. 3 lavages au TBS-BSA-saponine de 5 min chacun sont réalisés avant l'incubation avec l'anticorps secondaire GAM-cy3 (1/400) pendant 1 h. Suite à 2 lavages au TBS de 5 min, les noyaux sont marqués par incubation au Topro3 (1/1000) pendant 5 min. Enfin, les lamelles peuvent être montées sur lame après 3 derniers lavages au TBS de 5 min. Les lames sont observées au microscope confocal (Bio-Rad) grâce à une excitation au laser aux longueurs d'onde appropriées. Afin de différencier le marquage entre Cy3 et Topro3 la couleur de fluorescence de Topo3 a été changée en bleu (couleur dés noyaux).

### M3. Efflux de radiotraceurs

Les mesures de transport d'ions chlorure dans les cellules ont été réalisées à l'aide de la technique des efflux d'iodure radioactif (Becq et al., 1999 ; Dormer et al., 2001). Le traceur (¹²⁵I) est incorporé dans le milieu intracellulaire. Puis, la quantité de radiotraceur qui sort de la cellule est comptée après l'ajout de différents agents pharmacologiques. L'iodure est utilisé comme un traceur du transport d'ions chlorure. ¹²⁵I a de plus l'avantage d'avoir une courte durée de vie comparée à celle d'autres marqueurs comme ³⁵Cl (1/2 vie respective 30 jours et 300 000 ans).

Les cellules sont mises en culture sur des plaques 24 puits dans un milieu adéquat. 2 rinçages avec du milieu d'efflux (NaCl : 136,6 mM, KCl : 5,4 mM, KH₂PO₄ : 0,3 mM, NaH₂PO₄ : 0,3 mM, NaHCO₃ : 4,2 mM, CaCl₂ : 1,3 mM, MgCl₂ : 0,5 mM, MgSO₄ : 0,4 mM, HERPES : 10 mM, D-glucose : 5,6 mM) sont effectués afin d'éliminer les cellules mortes qui relarguent la radioactivité de façon anarchique. Puis, les cellules sont mises à incuber avec 500 µl de charge (1 µCi/ml de ¹²⁵INa) pendant 30 min pour les CHO-WT ou 1 h pour les CF15 et Calu-3. L'iodure s'équilibre de part et d'autre de la membrane cellulaire. Le robot (MultiPROBE, Packard) réalise les étapes suivantes : le milieu de charge est rincé avec du milieu d'efflux pour éliminer la radioactivité extracellulaire. Le surnageant est collecté toutes les minutes dans des tubes à hémolyse et le milieu est remplacé par un volume équivalent (500 µl). Les prélèvements des 3 premières minutes ne subissent pas l'addition de drogue, ils permettent d'obtenir une ligne de base stable, caractérisant la sortie passive des ions I. Les 7 prélèvements suivants sont obtenus en présence de la molécule à tester. A la fin de l'expérience, les cellules sont lysées par ajout de 500 µl de NaOH (0, 1 N) /0, 1 % SDS (30 min), ainsi, la radioactivité restée à l'intérieur de la cellule peut être déterminée. La radioactivité présente dans les tubes à hémolyse est comptée en coups par minute (cpm) grâce à un compteur gamma (Cobra II, Packard). Les résultats en cpm sont exprimés sous forme de vitesse de sortie d'iodure radioactif (R) d'après la formule suivante : R (min⁻¹) = [In(¹²⁵I t₁) - In(¹²⁵I t₂)]/(t₁-t₂) avec ¹²⁵I t₁ : cpm au temps t₁ ; ¹²⁵I t₂ : cpm au temps t₂. Ce flux d'iodure est représenté sous forme de courbe. Afin de quantifier la sortie d'iodure due à l'administration de la molécule testée, on calcule le flux relatif suivant qui permet de s'affranchir du flux de base : Vitesse relative (min⁻¹) =Rpic-Rbasal. Enfin, ces résultats sont normalisés pour pouvoir comparer l'effet des différentes drogues entre elles. Les résultats sont présentés sous la forme de moyenne +/- SEM. Le test statistique de Student est utilisé pour comparer l'effet des drogues aux contrôles (les valeurs correspondantes à P<0,01 sont considérées comme statistiquement significatives).

### M4. Test de cytotoxicité

Le test de toxicité au MTT est un test colorimétrique qui repose sur la capacité des déshydrogénases mitochondriales à métaboliser le MTT (sel de tétrazolium jaune) en formazan (pourpre). L'absorbance, proportionnelle à la concentration de colorant converti, peut être alors mesurée par spectrophotométrie. Les cellules sont mises à incuber sur des plaques 96 puits en présence de l'agent à tester pendant 2 h. 3 contrôles sont réalisés : 100 % cellules vivantes : cellules sans agent ; 0 % cellules vivantes : cellules laissées à l'air libre ; blanc : milieu sans cellule. Les cellules sont rincées avec du milieu RPMI sans rouge de phénol pour que la couleur du milieu n'interfère pas dans les mesures de l'absorbance. Puis, elles sont incubées pendant 4 h avec 100 µl de solution de RPMI supplémentée en MTT (0,5 mg/ml). Le milieu est alors éliminé, l'ajout de 100 µl de DMSO permet de solubiliser le colorant converti (formazan). L'absorbance est mesurée par spectrophotométrie à 570 nm (pourpre) ; 630 nm (bruit de fond). Afin de s'affranchir du bruit de fond, le calcul suivant est effectué : DO_{réelle}=DO₅₇₀ₙₘ-DO₆₃₀ₘₙ. Puis, les résultats sont normalisés par rapport aux contrôles (100 % et 0 % de cellules vivantes) et sont présentés sous forme de moyenne +/-SEM.

### RESULTATS

### R1. Effet de kenpaullone sur l'adressage de delF508 dans les cellules CF15

L'étude de l'adressage de la protéine delF508-CFTR est réalisée au laboratoire en combinant des approches de pharmacologie, d'imagerie cellulaire, des tests biochimiques et électrophysiologiques sur des cellules CF15 épithéliales humaines pulmonaires homozygotes pour la délétion delf508.

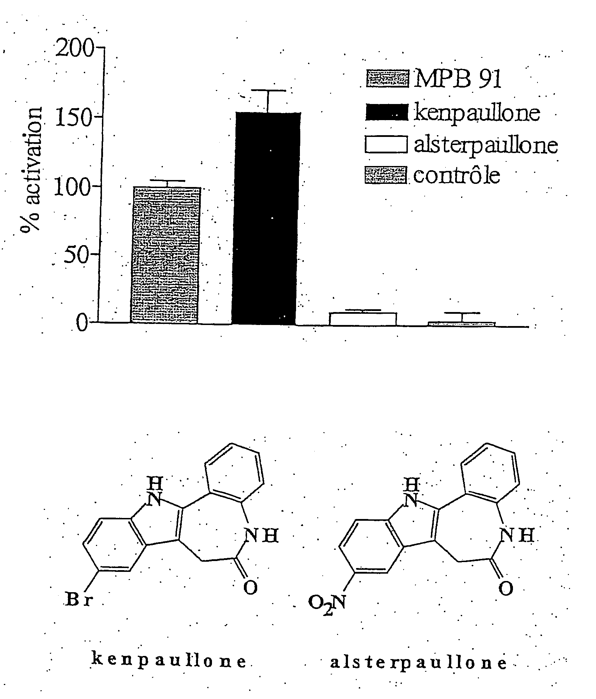

Pour chaque expérience, l'addition d'un cocktail (Forskoline 10 µM + Génistéine 30 µM) permet l'activation du CFTR quand celui-ci est localisé à la membrane. Ainsi, un efflux d'iodure pourra être observé si l'adressage de delF508 a été restauré. Les résultats, présentés sous forme d'histogramme ont été normalisés par rapport à un traitement de référence (traitement des cellules au MPB-91 250 µM pendant 2 h) pour lequel on considère qu'on a 100 % d'activité CFTR. La figure 1 montre qu'un traitement de 2 h avec 100 µM de kenpaullone restaure l'activité delF508-CFTR à un niveau supérieur de celui obtenu avec le MPB-91. En revanche, l'alsterpaullone (100 µM) dans les mêmes conditions, expérimentales que la kenpaullone n'a pas d'effet (figure 1). Ces résultats montrent que le traitement par la kenpaullone, mais pas l'alsterpaullone, des cellules CF15 pendant 2 h à 37°C restaure un adressage de la protéine delF508 et permet à celui-ci de fonctionner en tant que transporteur ionique (figure 1).

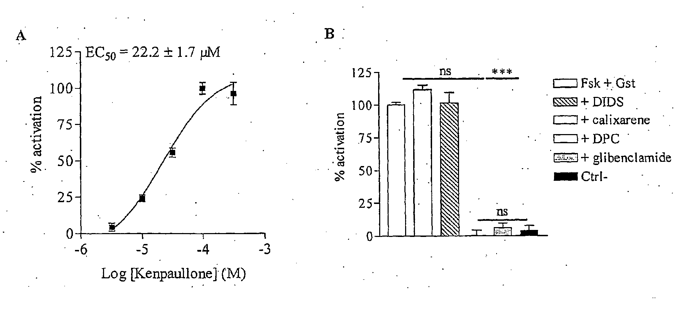

En l'absence de traitement des cellules, la protéine delF508 n'est pas membranaire et il n'y a pas d'efflux d'iodure stimulé par le cocktail (Forskoline 10 µM, Génistéine 30 µM). L'EC₅₀ (concentration de la molécule qui donne 50 % de l'efficacité maximale) de la kenpaullone a été déterminé à 22 ± 1,7 µM (figure 2A, n=4, pour chaque condition). Pour préciser l'effet observé, des inhibiteurs connus de CFTR sur l'activité de delF508 après traitement par la kenpaullone ont été testé. Les résultats présentés figure 2B montrent que ce transport est bloqué par le glibenclamide et le DPC mais insensible au DIDS et au calixarene. Ce profil pharmacologique correspond à celui de CFTR.

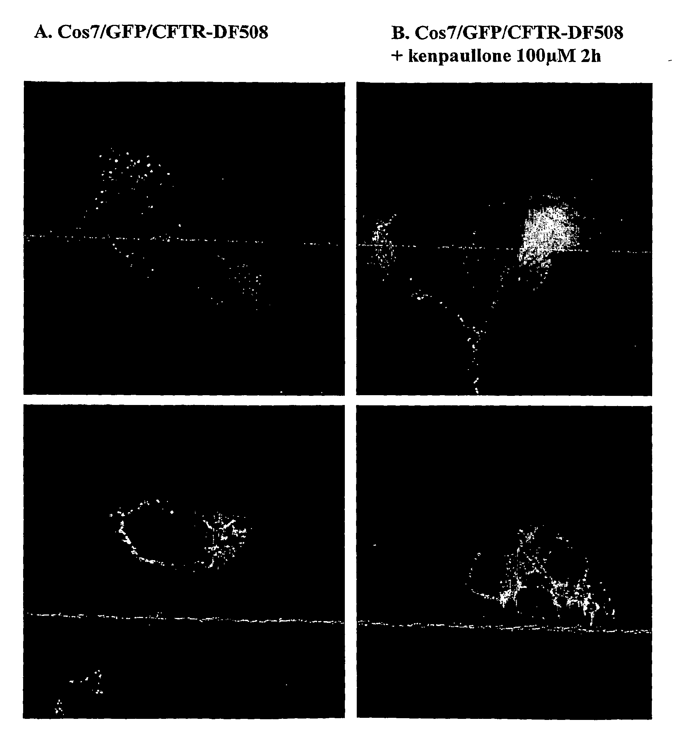

Chez les patients CF, la protéine delF508 est absente des membranes plasmiques du fait d'un mauvais adressage de la protéine qui est retenue dans le réticulum endoplasmique (RE). Par imagerie cellulaire dans les cellules CF15, delF508 est effectivement localisée dans des compartiments intracellulaires (figure 3A). En revanche le traitement avec 100 µM kenpaullonne permet de rediriger la protéine delF508 vers la membrane comme le présente la figure 3B.

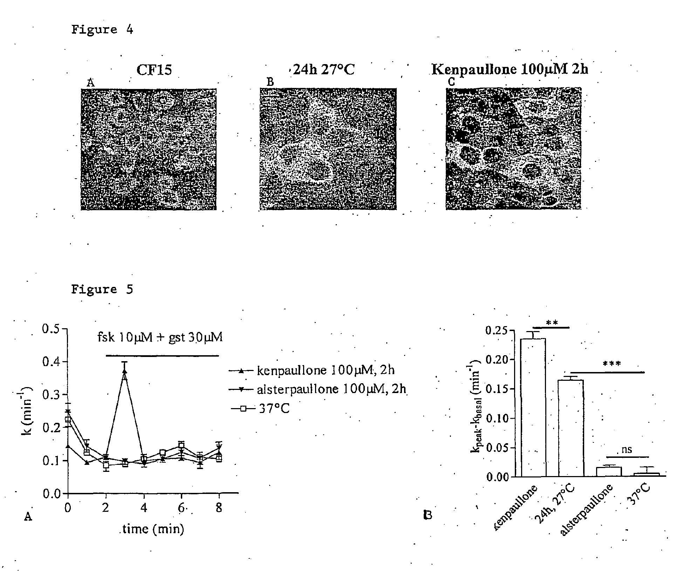

Les figures 4A à 4C illustrent également l'immunolocalisation de delF508-CFTR après 2 h de traitement par la kenpaullone ou en l'absence de traitement. Il s'agit d'une visualisation confocale de CFTR-delF508 dans des cellules CF15 avec un anticorps monoclonal anti-CFTR de souris. Les cellules CF15 traitées 24. h à 27°C ont. été utilisées comme témoin positif.

Les figures 5A et 5B représentent l'activation de delF508-CFTR dans des cellules CF15 après traitement par la kenpaullonne et à titre comparatif par l'alsterpaullone.

Les efflux d'iodure ont été observés après 2 h d'incubation avec 100 µM de ces composés ou en l'absence de traitement.

Les cellules CF15 ayant subi un traitement 24 h à 27°C ont été utilisées comme contrôle positif et les cellules CF15 comme contrôle négatif (37°C).

Le tableau ci-dessous donne un résumé d'expériences de compétition réalisées par la technique d'efflux d'iodure entre la kenpaullone et la machinerie de la chaperone du RE.

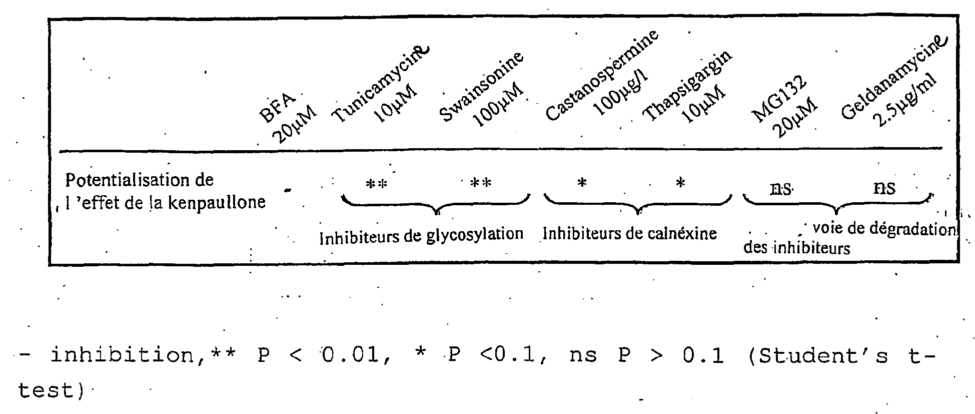

On observe une inhibition de la kenpaullone par la Brefeldine A (BFA), inhibiteur du trafic vésiculaire ERGIC, ce qui montre que la kenpaullone induit un réadressage de la protéine delF508-CFTR. Aucune modulation de l'effet de la kenpaullone n'est observée en présence de MG132, un inhibiteur de protéasome, ce qui montre une compétition entre la kenpaullone et MG132.

### R2. Effet, de la kenpaullonne sur l'activité de CFTR dans les cellules Calu-3

Afin de montrer que l'effet de la kenpaullonne est spécifique de l'adressage du delF508 et n'altère pas les autres canaux chlorure, la kenpaullone a été testée en tant qu'activateur potentiel sur des cellules Calu-3. Ces résultats ont été obtenus en efflux iodure sur cellules Calu-3. Nos contrôles sont la forskoline (5 µM, n=8) et le MPB-91 (250 µM, n=8) la kenpaullone (n=8) n'est pas un activateur du CFTR sauvage ni d'autres transports anioniques de ces cellules (pas de différence significative).

### R3. Effet de kenpaullone sur l'adressage de CFTR dans les cellules Calu-3

Afin de montrer que l'effet de la kenpaullonne est spécifique de l'adressage du delF508, la kenpaullone a été testée en tant que modulateur de l'adressage du CFTR sauvage sur des cellules Calu-3. Ces résultats ont été obtenus en efflux iodure sur cellules Calu-3 traitées 2 h par la kenpaullone (100 µM). L'activité CFTR dans cette condition expérimentale n'est pas significativement différente par rapport aux contrôles. Ces résultats démontrent que la kenpaullone n'affecte pas la voie d'adressage du CFTR sauvage ou d'autres canaux chlorure, ni n'altère l'activité de CFTR dans les cellules épithéliales humaines pulmonaires non-CF.

### R4. Cytotoxicité de la kenpaullone

Dans le but de tester la cytotoxicité de la kenpaullone, des cellules CHO-WT ont été incubées 2 h avec différentes concentrations en inhibiteurs avant de subir le test de viabilité cellulaire au MTT. Les résultats montrent que les cellules sont viables pour toutes les concentrations de la kenpaullone. Cette molécule ne présente donc pas de cytotoxicité cellulaire.

### CONCLUSIONS

Les tests d'efflux ont révélé que la kenpaullone permet une relocalisation membranaire de la protéine delF508-CFTR restaurant ainsi sa capacité de transport trans-membranaire, inhibe la voie de dégradation et apparaît moduler l'interaction entre CFTR et la calnéxine (mécanisme calcium-dépendant). Un traitement par la kenpaullone de cellules CF apparaît ainsi interférer avec la capacité que possède la machinerie de contrôle à interagir et à retenir la protéine F508del-CFTR dans le reticulum endoplasmique par l'intermédiaire d'une inhibition de la calnexine et des molécules chaperonnes qui interviennent dans la voie de dégradation.

Cette molécule n'est pas toxique dans les tests utilisés. De plus, des études de toxicité de la kenpaullone; réalisées chez l'animal, ont révélé une toxicité très faible. Une concentration plasmatique élevée peut être atteinte par couplage à PEG. La kenpaullone est considérée comme très sélective de l'inhibition de GSK-3.

### Exemple de formulation

On prépare une solution pour inhalation avec un nébuliseur ampoule à partir de chlorure de sodium, de chlorure de calcium déshydraté et d'eau pour préparations injectables.

La kenpaullone est ajoutée comme ingrédient actif.

La solution est formulée dans des ampoules de 2,5ml.

Des ampoules renfermant 5, 10 mg ou 20 mg kenpaullone sont ainsi préparées.

### BIBLIOGRAPHE

BECQ et al. (1999) Journal of Biological Chemistry 274, 27415-27425.
DORMER et al. (2001) Journal of Cell Science 114, 4073-4081.

## Revendications

1. Utilisation de dérivés de paullones pour fabriquer des médicaments pour le traitement de la mucoviscidose, ces dérivés répondant à la formule générale (II) :
dans laquelle
- X représente un groupe C = O, C-S-CH₃, C-S, -C-NHOH;
- R⁵ représente un atome d'hydrogène ou un groupe alkyle en C₁ à C₅,
- R¹² représente un atome d'hydrogène, ou un groupe -C-CO₂- (CH₃)₃, 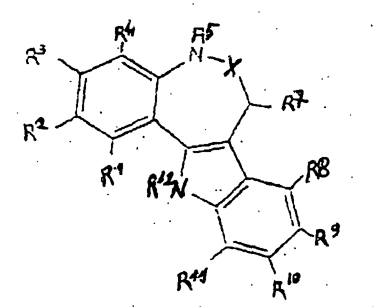
- R¹ à R¹, R⁷ à R¹¹, identiques ou différents, représentent un atome d'hydrogène, d'halogène (F, Cl, Br, I), In groupe hydroxy, alkylènehydroxy, alcynealkylènehydroxy, alcynehydroxycyclohexyle, alkyle, alkoxy, alkylènealkoxy, alkylènecyano, ces radicaux étant à chaîne droite ou ramifiée, an C1 à C18, le groupe alkylène étant saturé ou insaturé, Ladite chaîne étant Le cas échéant substituée par un ou plusieurs groupes hydroxy ou amino ; un groupe trifluorométhyle; un groupe -CCM; -COOM; ou -CH₂COOM, (avec M représentant un atome d'hydrogène, un groupe alkyle en C1 à C18, à chaîne droite ou ramifiée, substituée le cas échéant par un ou plusieurs groupes hydroxy et/ou amino) ; un groupe nitroso; ou un groupe cyano ;
et les sels physiologiquement acceptables de ces dérivés.

2. Utilisation selon la revendication 1, **caractérisée en ce que** X représente C = 0.

3. Utilisation pour fabriquer des médicaments pour le traitement de la mucoviscidose, de la kenpaullone répondant à la formule (V) :

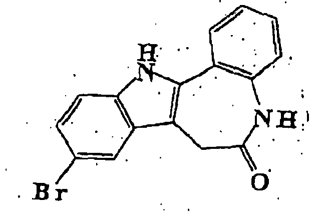

4. Utilisation selon l'une quelconque des revendications 1 à 3, **caractérisée en ce que** les médicaments sont préparés pour une administration sous formes de gélules, comprimés, dragées ou capsules.

5. Utilisation selon l'une quelconque des revendications 1 à 3, **caractérisée en ce que** les médicaments sont préparés pour une administration par voie injectable, sous forme de solution.

6. Utilisation selon l'une quelconque des revendications 1 à 3, **caractérisée en ce que** les médicaments sont préparés pour une administration sous forme d'aérosol.

## Claims

1. Use of paullone derivatives for the production of medicaments for the treatment of mucoviscidosis, the derivatives having formula (II): 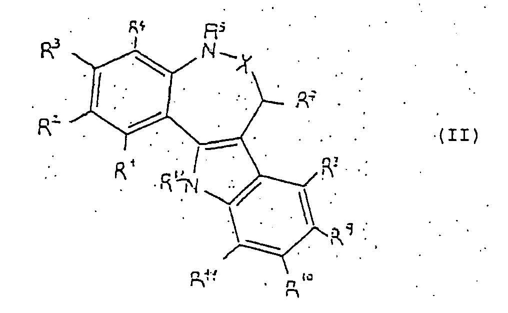 or physiologically acceptable salts thereof;
wherein
- X represents a C=O, C-S-CH₃, C-S or -C-NHOH group;
- R¹ to R⁴, R⁷ to R¹¹, identical or different, represent an atom of hydrogen, halogen (F, Cl, Br, I), a hydroxyl, alkylenehydroxyl, alkynalkylenehydroxyl, alkynhydroxycyclohexyl, alkyl, alkoxy, alkylenealkoxy, alkylenecyano group, these radicals having C₁ to C₁₈ linear or branched chains, the alkylene group being saturated or unsaturated; the said chain being substituted with one or more hydroxyl or amino groups as the case may be, a trifluoromethyl group, a -COM, -COOM or -CH₂COOM group (where M represents a hydrogen atom, a linear or branched C₁ to C₁₈ alkyl group, substituted with one or more hydroxyl and/or amino groups as the case may be), a nitroso group or a cyano group;
- R⁵ represents a hydrogen atom or a C₁ to C₅ alkyl group;
- R¹² represents a hydrogen atom or a -C-CO₂-(CH₃)₃ group.

2. Use according to claim 1, wherein X represents C=O.

3. Use of kenpaullone having the formula (V) for the production of medicaments for the treatment of mucoviscidosis:

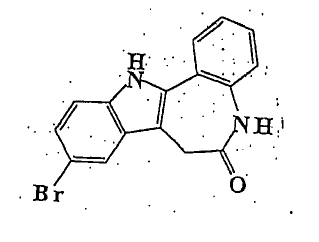

4. Use according to any one of claims 1 to 3, wherein the medicaments are prepared for administration in the form of gelatine capsules, tablets, sugar coated tablets or capsules.

5. Use according to any one of claims 1 to 3, wherein the medicaments are prepared for parenteral administration, in the form of a solution.

6. Use according to any one of claims 1 to 3, wherein the medicaments are prepared for administration in the form of an aerosol.

## Patentansprüche

1. Verwendung von Paullon-Derivaten zur Herstellung von Medikamenten zur Behandlung von Mukoviszidose, wobei die Derivate die Formel (II) haben: 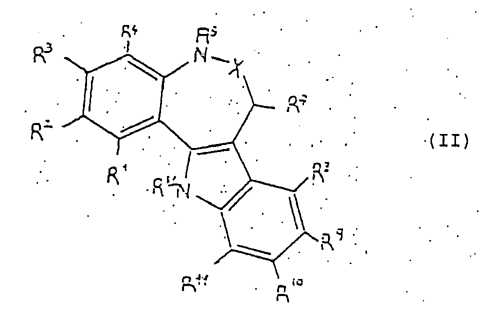 wobei
- X eine C=O, C-S-CH₃, C-S oder -C-NHOH Gruppe darstellt;
- identische oder unterschiedliche R¹ bis R⁴, R⁷ bis R¹¹ ein Wasserstoffatom, Halogenatom (F, Cl, Br, I), eine Hydroxy-, Alkylenhydroxy-, Alkynalkylenehydroxy-, Alkynhydroxycyclohexyl-, Alkyl-, Alkoxy-, Alkylenalkoxy-, Alkylenecyanogruppe darstellen, wobei diese Radikale eine lineare oder verzweigte C₁ bis C₁₈ Kette haben, wobei die Alkylengruppe gesättigt oder ungesättigt sein kann; wobei besagte Kette gegebenenfalls substituiert wird durch eine oder mehrere Hydroxy- oder Aminogruppen, eine Trifluoromethylgruppe, eine -COM, -COOM oder -CH₂COOM Gruppe (wobei M ein Wasserstoffatom, eine lineare oder verzweigte C₁ bis C₁₈ Alkylgruppe darstellt, die gegebenenfalls durch eine oder mehrere Hydroxy- und/oder Aminogruppen substituiert wird), eine Nitrosogruppe oder eine Cyanogruppe;
- R⁵ stellt ein Wasserstoffatom oder eine C₁ bis C₅ Alkylgruppe dar,
- R¹² stellt ein Wasserstoffatom oder eine -C-CO₂-(CH₃)₃ Gruppe dar,
und physiologisch akzeptable Salze davon.

2. Verwendung nach Anspruch 1, wobei X für C=O steht.

3. Verwendung von Kenpaullon gemäß der Formel (V) zur Herstellung von Medikamenten zur Behandlung von Mukoviszidose:

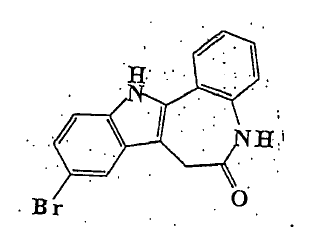

4. Verwendung nach einem beliebigen der Ansprüche 1 bis 3, wobei die Medikamente zur Verabreichung in Form von Gelatinekapseln, Tabletten, Dragées oder Kapseln hergestellt werden.

5. Verwendung nach einem beliebigen der Ansprüche 1 bis 3, wobei die Medikamente zur parenteralen Verabreichung in Form einer Lösung hergestellt werden.

6. Verwendung nach einem beliebigen der Ansprüche 1 bis 3, wobei die Medikamente zur Verabreichung in Form eines Aerosols hergestellt werden.
